(19) Europäisches Patentamt
European Patent Office
Office européen des brevets

(11) Publication number: **0 428 109 A2**

# (12) EUROPEAN PATENT APPLICATION

(21) Application number: 90121636.6

(22) Date of filing: 12.11.90

(51) Int. Cl.[5]: C07H 19/19

(30) Priority: 13.11.89 US 435852

(43) Date of publication of application: 22.05.91 Bulletin 91/21

(84) Designated Contracting States: AT BE CH DE DK ES FR GB GR IT LI LU NL SE

(71) Applicant: Bristol-Myers Squibb Company
345 Park Avenue
New York, N.Y. 10154(US)

(72) Inventor: Vemishetti, Purushotham
507 Tilden Drive
East Syracuse, New York 13057(US)
Inventor: Howell, Henry G.
4114 Gates Road North
Jamesville, New York 13078(US)
Inventor: Walker, Donald G.
8423 Lace Bark Lane
Liverpool, New York 13207(US)
Inventor: Brodfuehrer, Paul R.
108 Wellesley Road
Syracuse , New York 13207(US)
Inventor: Shih, Kun-Mao
5811 Coventry Road
East Syracuse, New York 13057(US)

(74) Representative: Kinzebach, Werner, Dr. et al
Patentanwälte Reitstötter, Kinzebach und Partner Sternwartstrasse 4 Postfach 86 06 49
W-8000 München 86(DE)

## (54) Deoxyfluoronucleoside process.

(57) There is disclosed a process for synthesizing 2′-fluoro-2′,3′-dideoxyarabinofuranose derivatives of inosine and adenine on a large scale which involves coupling of a fluorosugar derivative and a purine reactant to provide a purine nucleoside intermediate which is then deoxygenated. 6-Chloro or 6-benzamidopurine and 1,3,5-tri-O-benzoyl-2-deoxy-2-fluoroarabinofuranose are used as starting materials.

EP 0 428 109 A2

# DEOXYFLUORONUCLEOSIDE PROCESS

## Field of the Invention

The invention provides a new chemical process for preparing the nitrogen containing carbohydrate derivatives FddaraA and FddaraI having Formulas VIa and VIb herein. (Class 536, subclass 55.3). The latter are antivirally active N-glycosides having plural nitrogen containing hetero rings, viz. the purine ring (Class 536, subclass 24).

## Description of Related Art

Nucleoside derivatives containing a 2′-fluoroarabino substituent have been known for a number of years. 2′,3′-Dideoxy-2′-fluoroarabinonucleoside analogs, including the adenosine and inosine analogs, have been shown to display significant antiviral activity against HIV (Marquez, et al., Biochem. Pharmacol., 1987, 36, 2719, and European Patent Application 287,313 published Oct. 19, 1988; Herdewijn, et al., J. Med. Chem., 1987, 30, 2131; Pauwels, et al., Biochem. Pharmacol., 1988, 37, 1317). The introduction of the fluorine atom renders the compounds more stable to aqueous acid than the unfluorinated compounds, (Marquez, et al.,loc. cit.) and resistant to purine and pyrimidine phosphorylases (Codere, et al., J. Med. Chem, 1983, 26, 1149; Chou, et al., Cancer Research, 1981, 41, 3336; Stoeckler, et al., Biochem. Pharmacol., 1982, 31, 1723).

The major synthetic challenge for the large scale synthesis of these compounds is the introduction of the 2′-fluoro substituent having the arabino configuration in high yield. Attempts to introduce the fluorine substituent into an unfluorinated nucleoside intermediate have either been unsuccessful or very inefficient. Attempts to prepare FddaraA (Formula VIa below) having the arabinofuranose configuration by displacement with inversion of the 2′-triflyloxyribonucleoside with fluoride led to elimination products (Marquez, et al., loc. cit.). A similar displacement using the 2′-triflyloxyarabinonucleoside produced ribofuranose compounds isomeric with those required by the present invention in 50% yield (Fribo A having the undesired ribofuranose configuration) and 30% of elimination products (Ranganathan, Tetrahedron Letters, 1977, 1291). 9-(2′,3′-Dideoxy-2′-fluoroarabinofuranosyl)adenine has been produced by treatment of 3′-deoxy-5′-trityloxyadenosine (cordecypin) with diethylamminosulfur trifluoride (DAST) but in only 10% yield (Heredjwin, et al., loc. cit.). Thus, state of the art with respect to this approach is that the elimination reaction is a serious drawback, and even when it is avoided or minimized, the displacement reaction to produce the fluorine compound is not an efficient transformation.

A second approach is to employ a suitable fluorine substituted arabinose derivative for coupling with the purine base. Several such coupling reactions with various fluorosugars have been reported previously. 5-O-Benzyl-2-deoxy-1,3-di-O-acetyl-2-fluoroarabinose has been coupled with 2,6-dichloro purine under fusion conditions to furnish the desired 2′-fluoroarabinonucleoside intermediate in 27% yield (Wright, et al., J. Org. Chem., 1969, 34, 2632). Further transformation of this intermediate to 2′-deoxy-2′-fluoroarabinoadenosine took place in 14% yield. $N^6$-Benzoyladenine has been coupled with 3-O-acetyl-5-O-benzoyl-1-bromo-2-deoxy-2-fluoro-α-D-arabino furanose to afford the desired coupling product in 34% yield (Chu, et al., Chem. Pharm. Bulletin, 1989, 37, 336). Hydrolysis of the various acyl groups afforded the desired adenosine derivative in 27% overall yield. 2-Deoxy-2-fluoro-1,3,5-tri-O-benzyl arabinofuranose has been used, after conversion to a 1-halide, in coupling reactions with pyrimidine bases to provide nucleoside analogs, and the method for its production can be carried out on a large scale (Howell, et al., J. Org. Chem. 1988, 53, 85-88, and Brundidge, et al., U. S. Patent No. 4,625,020, patented November 25, 1986). This is the starting material which is employed in the present process.

## Summary of the Invention

The process of the present invention is directed to an efficient process suitable for large scale use for preparing FddaraA (Formula VIa) and FddaraI (Formula VIb). It is represented by reaction Steps a-g shown in the following reaction scheme.

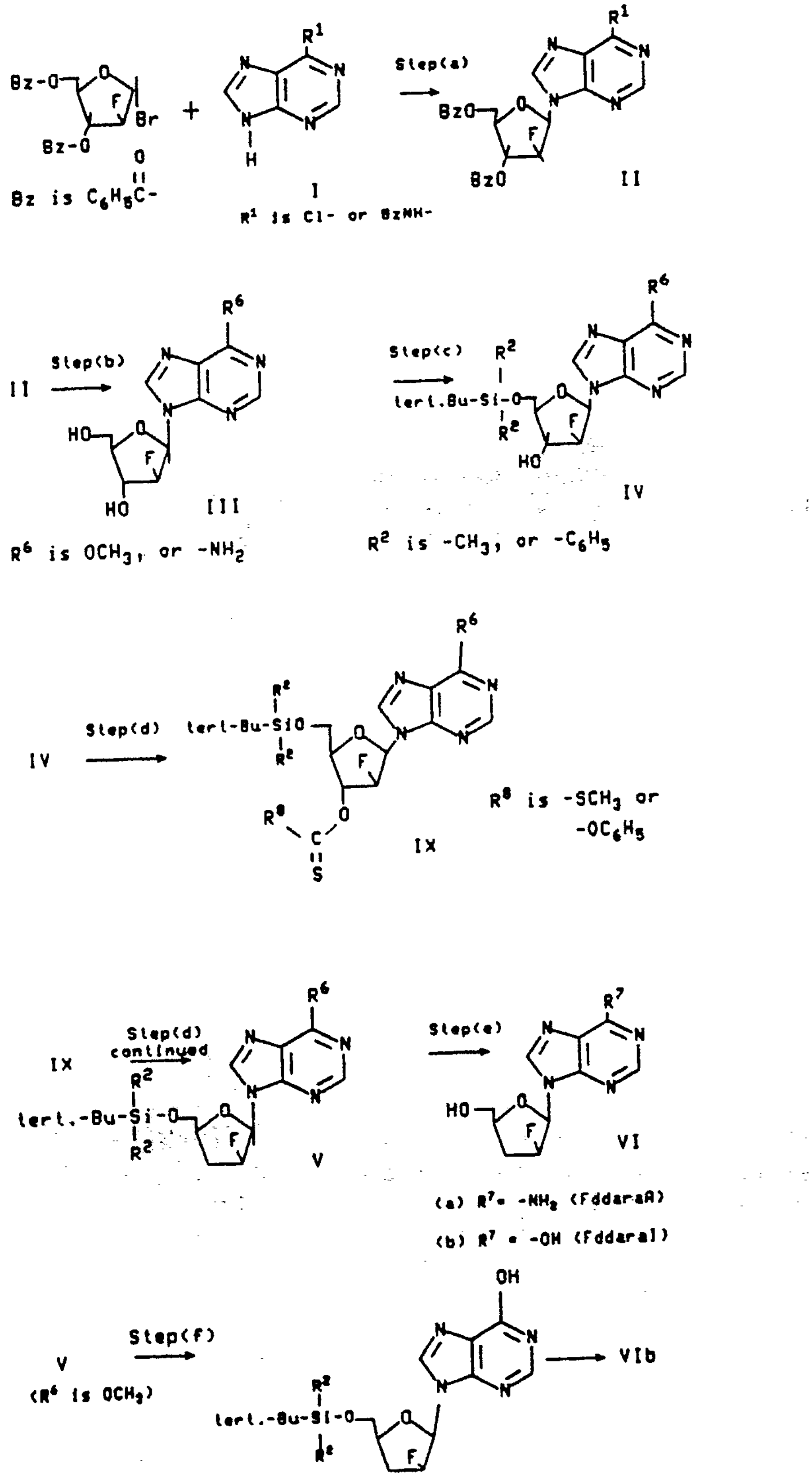
Bz is $C_6H_5\overset{O}{\overset{\|}{C}}$-
I
$R^1$ is Cl- or BzNH-
Step(a)
II
Step(b)
III
$R^6$ is $OCH_3$, or $-NH_2$
Step(c)
IV
$R^2$ is $-CH_3$, or $-C_6H_5$
Step(d)
IX
$R^8$ is $-SCH_3$ or $-OC_6H_5$
Step(d) continued
V
Step(e)
VI
(a) $R^7$ = $-NH_2$ (FddaraA)
(b) $R^7$ = -OH (Fddaral)
Step(f)
($R^6$ is $OCH_3$)
VIb

II
($R^1$ is Cl)

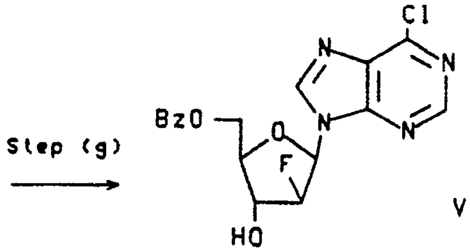


VII

VII

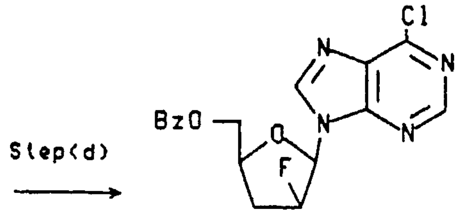


VIII

Steps (a) and (b) wherein $R^1$ is the benzoylamino are similar to the process described by Chou, et al., except that 1-bromo-2-deoxy-3,5-di-O-benzoyl-2-fluoro-α-D-arabinofuranose, referred to herein as the "fluorosugar" is used as starting material. The preparation of this material is described in U. S. Patent No. 4,625,020 cited above. The alternative use of 6-chloropurine in this process (Step (a) $R^1$ is Cl) as starting material for the preparation of FddaraI (Formula VIb) is unique as a means for introducing the 6-OH of this inosine-related product. This occurs in Step (b) by methanolysis to yield the 6-methoxy compound which is then cleaved in Step (e) or (f) to the hydroxy compound.

The method of deoxygenation in Step (d) is also unique in that the methyl xanthate ester is substituted for the phenoxythiocarbonate ester which is ordinarily employed in reductive deoxygenations of this type. Using this method FddaraI (VIb) is produced in six steps from the fluorosugar and 6-chloropurine in an overall yield of 30% which is far superior to any method reported in the prior art. In the production of FddaraA (VIa) the product is produced from the fluorosugar and 6-benzoylaminopurine in six steps in 35% overall yield, also representing a significant improvement over published methods.

Step (g) offers a variation of Step (b) which eliminates the need for the 5′-OH blocking reaction of Step (c). Step (g) is a selective hydrolysis of II to afford intermediates VII which can be substituted for IV and V in Steps (d) and (e), respectively, to provide VIII which can be carried through steps similar to the process steps shown to provide FddaraI. The intermediate of Formula VIII on reaction with ammonia yields FddaraA.

## Detailed Description of the Invention

Step (a): Coupling of the fluorosugar, 1-bromo-2-deoxy-3,5-di-O-benzoyl-2-fluoro-α-D-arabinofuranose, with 6-chloropurine as purine reactant is best carried out by first forming the sodium salt of the purine reactant and then carrying out the coupling reaction under conditions which favor $S_n2$ displacement and result in formation of the nucleoside intermediate as the desired β anomer (Procedure 1). In the present instance, with 1:1 acetonitrile methylene chloride as reaction solvent and sodium hydride to form the sodium salt of the purine reactant, the β/α-anomer ratio of the product determined *in situ* was 10/1, and the isolated yield employing ethanol for separation of the isomers was 73% of the pure β anomer. When the coupling is carried out as has been described in the prior art using the silylated purine reactant rather than the sodium salt thereof, the anomers were produced in a 3.4/1 ratio (β/α) and the β-anomer was recovered in 34% yield. Refer to U. S. Patent 4,625,020 cited above at column 9 for representative reaction conditions. Various solvents were examined for use in this process including tetrahydrofuran, dioxane, diglyme, chloroform, ethyl acetate, and acetonitrile but a 1:1 mixture of acetonitrile and methylene chloride was found to be the preferred solvent. The reaction can be conducted at room temperature, about 36 hours is required for completion, or heated at reflux for two to three hours. Chromatography is not required for purification of the intermediate thus particularly adapting the process for large scale use.

When using $N^6$-benzoyladenine as purine reactant, the pure β-anomer of the nucleoside intermediate produced could not be crystallized in pure form as above (Procedure 9), and the 15/1 β/α anomeric mixture

was subjected directly to methanolysis in Step (b) whereupon the pure β-anomeric product was isolated in 57% overall yield for the two steps.

Step (b): The methanolysis reaction in Step (b) is carried out using freshly prepared sodium methoxide in methanol, and has the purpose of removing the benzoyl groups which esterify the hydroxyl groups of the fluorosugar moiety of the nucleoside intermediate. In the case of the synthesis of FddaraI from 6-chloropurine, the methanolysis step also results in replacement of the 6-chlorine atom with the methoxy group conveniently introducing the oxygen atom required for the inosine derivative which is one object compound of the process (Procedure 2). Both reactions take place simultaneously, and the desired 6-methoxy purine nucleoside intermediate is obtained in 71% yield. In the case of methanolysis of the 6-benzoylamino purine nucleoside intermediate the N-benzoyl group is removed in addition to removal of the benzoyl groups in the fluorosugar moiety (Procedure 10).

Step (c): For reductive deoxygenation of the 3′-hydroxyl group of the fluorosugar nucleoside intermediate in Step (d), the 5′-hydroxy group is first protected by conversion to the tert-butyldimethylsilyl or tert-butyldiphenylsilyl ether in Step (c). This takes place readily and in high yield using tert.-butyldimethylsilyl chloride or tert-butyldiphenylsilyl chloride, and imidazole in dimethylformamide (Procedures 3, 11, and 12). The tertiary butyldimethylsilyl ether is preferred because a higher yield in the subsequent deoxygenation step is obtained when it is used.

Step (d): Conventionally reductive deoxygenation of the type employed in this process is carried out by formation of the phenoxythiocarbonate ester and treatment thereof with tri-n-butyltin hydride and azobisisobutyronitrile. This method is not, however, desirable for large scale operation because the yields are lower than is desirable, and the phenylthionochloroformate required as reactant is an expensive reagent. Accordingly the present process was developed according to which the methyl xanthate ester is formed at the 3′-hydroxyl group. This takes place in approximately 90% yield when using carbon disulfide, sodium hydroxide, and methyl iodide. The xanthate ester may be reduced without purification by treatment thereof with tri-n-butyltin hydride and azobisisobutyronitrile employing toluene as solvent. The deoxygenation step is thus accomplished in 78% yield with respect to the 6-methoxypurine nucleoside intermediate, and in 82% yield in the case of the 6-aminopurine nucleoside intermediate (Procedures 4, 5, 13, 14, 15, 16, 20, and 21).

Step (e): The final steps of the process involve removal of the 5′-silyl ether protecting group, and when $R^6$ is methoxy, conversion of the latter to -OH. Removal of the 5′-silyl ether takes place in conventional fashion, a convenient method being treatment with tetra-n-butylammonium fluoride in tetrahydrofuran as solvent (Procedure 17). For hydrolysis of the 6-methoxy and 5′-silyloxy groups in the preparation of FddaraI, heating with aqueous sodium hydroxide may be employed. A temperature of 75° C is satisfactory. This results in cleavage of both the methoxy group, and in removal of the silyl ether protecting group (Procedure 8).

Step (f): A preferred means of operation in preparing FddaraI, however, is to employ separate reactions to cleave the methyl group and the silyl ether group. Treatment with trimethylsilyl bromide or trimethylsilyl iodide is preferred for conversion of the 6-methoxy or 6-OH (Procedure 6). The tertiary butyldimethylsilyl group is removed with tetra-n-butylammonium fluoride as above (Procedure 7). In preparing FddaraI by the one step aqueous sodium hydroxide method, a 73% yield of the product is obtained. However, column chromatography with activated carbon and subsequently with silica gel is necessary for the final purification and this method is therefore less preferred than the stepwise removal sequence. It is preferred to first convert the 6-MeO to 6-OH , and to then hydrolyze the 5-O-tert-butyldimethylsilyl group. According to the stepwise mode, the overall yield is 75% and only one chromatographic step is required.

The present method applied to the production of FddaraI involves six steps and provides the product in 30% overall yield. For the production of FddaraA this method involves a six step synthesis and a 35% overall yield. Both are amenable to large scale production.

FddaraI can be prepared from FddaraA by enzymatic hydrolysis using the enzyme adenosine deaminase (Procedure 18). This transformation takes place at approximately room temperature or slightly above (36° C) in aqueous solution.

A further feature of the present invention involves selective hydrolysis of the 3′-O-benzoyl ester group of intermediate II produced in Step (a) when $R^1$ is Cl to provide a mono-5-O-benzoyl ester of Formula VII. The latter can be deoxygenated as in Step(d) to provide 9-(5-O-benzoyl-2,3-dideoxy-2-fluoro-β-D-arabinofuranosyl)-6-chloropurine of Formula VIII. This is illustrated in Procedures 19 and 20. The intermediate of Formula VIII is adapted for transformation into either FddaraI or FddaraA. For instance, intermediate VIII on reaction with saturated methanolic ammonia in a pressure vessel at 83° C yields FddaraA (Procedure 22). Alternatively, treatment of intermediate VIII with sodium hydroxide yields FddaraI (Procedure 23).

Description of Specific Embodiments

Melting points were taken on a Thomas-Hoover capillary melting point apparatus and are not corrected. Proton NMR spectra were recorded at 360 MHz using a Bruker AM-360 NMR and chemical shifts are expressed in ppm relative to TMS.

Abbreviations:
Bz benzoyl
DMF dimethylformamide
DMSO dimethylsulfoxide
EtOH ethanol
EtOAC ethyl acetate
HPLC high performance liquid chromatography
MeOH methanol
NMR nuclear magnetic resonance
THF tetrahydrofuran
TMS tetramethylsilane

Procedure 1:

9-(2-deoxy-3,5-di-O-benzoyl-2-fluoro-β-D-arabinofuranosyl-6-chloropurine.

A 5 L flask was flushed with dry $N_2$ and charged with 6-chloropurine (62.0 g, 0.40 mol) and dry $CH_3CN$ (700 mL). The mixture was stirred under a blanket of $N_2$ and 80% NaH-mineral oil dispersion (12.6, 0.40 mol) was added in four equal portions. The mixture was warmed and stirred at 55°C until the evolution of $H_2$ was complete (0.5 to 1 h). A solution of 1-bromo-2-deoxy-3,5-di-O-benzoyl-2-fluoro-α-D-arabinofuranose (166 g, 0.39 mol) in $CH_2Cl_2$ (700 mL) was added from a dropping funnel at a rapid rate. After 3 h, an HPLC analysis of the reaction indicated the bromosugar was consumed. The reaction mixture was filtered and the filtrate evaporated. The residue was partitioned between EtOAc (1500 mL) and $H_2O$ (500 mL) the organic layer removed, and the aqueous extracted once with EtOAc (500 mL). The combined organics (2.5 L) were washed with $H_2O$ (2 x 300 mL) and brine (2 x 400 mL), dried ($Na_2SO_4$) and filtered. The filtrate was concentrated in vacuo and the residue evaporated from abs. EtOH (500 mL), diluted with $CH_2Cl_2$ (100 mL) and abs. EtOH (500 mL). The mixture was stirred at 20°C for 1 h and cooled to 0-5°C for 2 h, before collecting the product as a light yellow crystalline solid. The material was dried under high vacuum to constant weight giving 154.5 g (73%) of the desired product as an EtOH solvate. (HPLC analysis prior to isolation, indicated the N-9 β/α isomer ratio was about 10/1, with small amounts of isomers resulting from alkylation at the N7 position of the purine also present. However, the final isolated product was pure, containing none of the other isomers).

$^1$H NMR ($CDCl_3$) δ 1.2 (t, 3H, Me), 1.4 (br s, 1H, OH), 3.7 (q, 2H, $CH_2$), (these 3 peaks represented 1 mol of EtOH per mol of product), 4.6 (m, 1H, C4′), 4.85 (d, 2H, C5′), 5.35 (dd, 1H, C2′), 5.75 (dd, 1H, C3′), 6.7 (dd, 1H, C1′), 7.4-8.2 (m, 10H, ArH), 8.4 (d, 1H, C8), 8.8 (s, 1H, C2).

Procedure 2:

9-(2-deoxy-2-fluoro-β-D-arabinofuranosyl)-6-methoxypurine.

A solution from sodium metal (7.4 g, 0.32 mol) and MeOH (1 L) was stirred at 20°C in an inert atmosphere ($N_2$) and 6-chloro-9-(2-deoxy-2-fluoro-3,5-di-O-benzoyl-β-D-arabinofuranosyl)purine (140 g, 0.28 mol) added. The resulting mixture was heated at reflux for 1/2 h at which time HPLC analysis indicated the starting material was consumed. The solution was cooled to ca 20°C and concentrated in vacuo. The resulting residue was dissolved in $H_2O$ (500 mL) and extracted twice with $CH_2Cl_2$ (350 mL). The pH of the aqueous was adjusted to 7.0 using 6 N HCl. The solution was concentrated to approximately 250 mL and the product slurry stored for 6 h at 5°C. The product was collected, washed ($H_2O$, 2 x 50 mL), and the pale yellow crystals dried under high vacuum giving 57 g (71%) of the desired product.

$^1$H NMR (DMSO-$d_6$) δ 3.63 (m, 2H, C5′), 3.9 (q, 1H, C4′), 4.1 (s, 3H, $OCH_3$), 4.45 (dt, 1H, C3′), 5.25 (dt, 1H,

C2'), 6.1 (s, 1H, OH), 6.5 (dd, 1H, Cl'), 8.5 (d, 1H, C8), 8.6 (s, 1H, C2).

Procedure 3:

9-(5-O-tert-Butyldimethylsilyl-2-deoxy-2-fluoro-β-D-arabinofuranosyl)-6-methoxypurine.

A mixture of DMF (320 mL) and 3 A molecular sieves (40 g) was stirred with 9-(2-deoxy-2-fluoro-β-D-arabinofuranosyl)-6-methoxypurine (54.0 g, 0.190 mol) for 2 h. Imidazole (22.44 g, 0.42 mol) and tert-butyldimethylsilyl chloride (28.6 g, 0.19 mol) were added. HPLC was used to monitor the reaction, and portions of tert-butyldimethylsilyl chloride were added until complete conversion was obtained (total silyl chloride used 54.64 g, 0.296 mol, 1.56 equiv). The mixture was diluted with MeOH (80 mL), filtered, poured into $H_2O$ (1 L) and extracted with EtOAc (3 x 350 ml). The combined extracts (1.15 L) were washed with $H_2O$ (2 x 300 mL) and brine (1 x 250 mL), dried ($MgSO_4$) and evaporated to give 84.1 g of the product as an oil. The pure product was obtained by flash chromatography on silica gel, eluting with 25% EtOAc-hexane (which gave fractions containing the 3,5-di-tert-butyldimethylsilylated product, 1 to 2 %), and then with 50% EtOAc-hexane (which eluted the desired product). The product fractions were pooled and evaporated giving 64.7 g (86%) of the pure crystalline product.
$^1H$ NMR ($CDCl_3$) δ 0.11 (s, 3H, SiMe), 0.23 (s, 3H, SiMe), 0.9 (s, 9H, $SiCMe_3$), 2.8 (br s, 1H, OH), 3.85 (m, 2H, C5'), 4.05 (m, 1H, C4'), 4.15 (s, 3H, MeO), 4.65 (dt, 1H, C3'), 5.15 (dt, 1H, C2'), 6.57 (dd, 1H, Cl'), 8.2 (d, 1H, C8), 8.5 (s, 1H, C2).

Procedure 4:

9-(5-O-tert-Butyldimethylsilyl-2-deoxy-2-fluoro-3-O-methylthiothionocarbonyl-β-D-arabinofuranosyl)-6-methoxypurine.

A solution of 9-(5-O-tert-Butyldimethylsilyl-2-deoxy-2-fluoro-β-D-arabinofuranosyl)-6-methoxypurine (6.74 g, 16.7 mmol) and $CS_2$ (2.0 mL, 33.8 mmol) in DMSO (34 mL) was stirred and cooled to 15°C. A solution of 5 M NaOH (3.7 mL, 18.6 mmol) was slowly added dropwise maintaining temperature at 15°C. After 1/2 h, MeI (1.16 mL, 18.6 mmol) was added to the red-orange solution and the temperature rose to 23°C while the color nearly dissappeared. After 1 h, the reaction was diluted with $H_2O$ (150 mL) and extracted with EtOAc (2 x 200 mL). The combined organics (ca. 500 mL) were washed with $H_2O$ (5 x 50 mL), brine (50 mL), dried ($MgSO_4$), filtered and evaporated to a residue. The product crystallized upon extended drying in vacuo giving 8.3 g (98%). The pure material was obtained by flash chromatography on silica gel, eluting with 10-20% EtOAc-hexane to give 7.7 g (93%) of the xanthate: mp 84.5-85.5°C (dec.), $^1H$ NMR ($CDCl_3$) δ 0.12 (2s, 6H, $Si(Me)_2$), 0.94 (s, 9H, $SiC(Me)_3$), 2.62 (s, 3H, SMe), 4.0 (m, 2H, C5') 4.21 (s, 3H, MeO), 4.31 (m, 1H, C4'), 5.27 (dd, 1H, C2'), 6.37 (dd, 1H, C3'), 6.6 (dd, 1H, C1'), 8.25 (d, 1H, C8), 8.55 (s, 1H, C2).

Procedure 5:

9-(5-O-tert-Butyldimethylsilyl-2,3-dideoxy-2-fluoro-β-D-arabinofuranosyl)-6-methoxypurine.

Toluene (100 mL) was gassed with $N_2$ for 30 min, then placed under vacuum for 30 min and gassed again for 30 min with $N_2$ to remove oxygen. The 9-(5-O-tert-butyldimethylsilyl-2-deoxy-2-fluoro-3-O-methylthiothionocarbonyl-β-D-arabinofuranosyl)-6-methoxypurine (7.7 g, 16 mmol) in toluene (50 mL) was added and the solution again deoxygenated by treating with $N_2$, vacuum and $N_2$. The solution was stirred and 2,2'-azobisisobutyronitrile (.52 g, 3.15 mmol) was added, followed again by 15 min of $N_2$ degassing. Tri-n-butyltin hydride (5.7 mL, 20.5 mmol) was added dropwise and the reaction heated at 80°C for 25 min. HPLC analysis indicated the reaction was complete and the solvent was evaporated to a semi-solid residue. The pure product was obtained by flash chromatography on silica gel eluting with 10-40% EtOAc-hexane to give 5.5 g (91%) of the product as colorless crystals.

$^1H$ NMR ($CDCl_3$) δ 0.08 (s, 3H, SiMe), 0.09 (s, 3H, SiMe), 0.9 (s, 9H, Si(Me)$_3$), 2.43-2.61 (m,2H, C3′), 3.82 (dd, 2H, C5′) 4.16 (s, 3H, MeO), 4.18-4.28 (m, 1H, C4′), 5.28 (ddt, 1H, C2′), 6.35 (dd, 1H, C1′), 8.26 (d, 1H, C8), 8.49 (s, 1H, C2).

Procedure 6:

9-(5-O-tert-Butyldimethylsilyl-2,3-dideoxy-2-fluoro-β-D-arabinofuranosyl)hypoxanthine.

A solution of 9-(5-O-tert-butyldimethylsilyl-2,3-dideoxy-2-fluoro-β-D-arabinofuranosyl)-6-methoxypurine (23.7 g, 0.62 mol) and dry $CH_3CN$ (300 mL) was stirred at 20°C. Trimethylsilyl iodide (9.3 mL, .0653 mol) was added over 2 min. Since HPLC analysis after 16 h indicated some starting material remained (ca 5%), additional trimethylsilyl iodide (1 mL, .007 mol) was added and the reaction warmed at 50°C for 30 min., at which time HPLC indicated the reaction was complete. After cooling to 20°C, the pH was adjusted to 7.0 by the addition of 30 mL of saturated $NaHCO_3$. The solvent was evaporated under vacuum and the residue partitioned with $H_2O$ (100 mL) and EtOAc (400 mL). The organic layer was washed with 5% $NaHSO_3$ (50 mL) and the combined aqueous layers were extracted with EtOAc (1 x 100 mL). The combined organic layers were dried ($MgSO_4$) and concentrated to a white crystalline solid, 21.4 g (94%); $^1H$ NMR ($CDCl_3$) δ .05 (2s, 6H, Si(Me)$_2$), 0.9 (s, 9H, SiC(Me)$_3$), 2.5 (m, 2H, C3′), 3.8 (m, 2H, C5′), 4.27 (m, 1H, C4′), 5.25 (m, 1H, C2′), 6.25 (dd, 1H, Cl′), 8.2 (m, 2H, C2, C8).

Procedure 7:

9-(2,3-Dideoxy-2-fluoro-β-D-arabinofuranosyl)hypoxanthine. (Fddaral)

To a solution of 9-(5-O-tert-butyldimethylsilyl-2,3-dideoxy-2-fluoro-β-D-arabinofuranosyl)hypoxanthine (20.9 g, .057 mol) in THF (300 mL), tetra-n-butylammonium fluoride (57 mL, .057 mol) was added as a 1 M solution in THF. HPLC indicated the complete consumption of the starting material in 3h. The solvent was evaporated to a residue. The pure product was obtained by flash chromatography on silica gel,(eluting with 5% MeOH-$CH_2Cl_2$) as a white crystalline solid by evaporating the approproate chromatography fractions. The weight of Fddl obtained was 11.68 g (80%). $^1H$ NMR ($D_2O$) δ 2.4 (m, 1H, C3′), 2.75 (m, 1H, C3′), 3.9 (m, 2H, C5′), 4.5 (m, 1H, C4′), 5.5 (ddt, 1H, C2′), 6.4 (dd, Cl′), 8.2 (s, 1H, C8), 8.4 (s, 1H, C2).

Procedure 8:

9-(2,3-Dideoxy-2-fluoro-β-D-arabinofuranosyl)hypoxanthine (Fddaral) from 9-(5-O-tert-butyldimethylsilyl-2,3-dideoxy-2-fluoro-β-D-arabinofuranosyl)-6-methoxypurine.

A solution of MeOH (250 mL, 50% aqueous) and 9-(5-O-tert-butyldimethylsilyl-2,3-dideoxy-2-fluoro-β-D-arabinofuranosyl)-6-methoxypurine(21.4 g, .056 mol) was stirred and 2 N NaOH (41.9 mL, .083 mol) was added. After stirring at 75°C for 18 h the dark solution was cooled and the pH adjusted to 7.0 with Dowex ion-exchange resin (50x 8-100). After filtration, the solvent was evaporated to give the crude product which was purified by chromatography; first, a column of 14-60 mesh granular activated charcoal was eluted with 1 L water (discarded), then EtOH - $H_2O$ (50/50, 3 L, containing 10% $NH_4OH$) to give the product fraction, which was then chromatographed on a column of silica gel (70-200 mesh)and eluted with 15% MeOH-$CH_2Cl_2$ to give 10.4 g (73% yield). This material was lyophylized to give the product as a white amorphorus power. (NMR identical to that above).

Procedure 9:

9-(2-Deoxy-3,5-di-O-benzoyl-2-fluoro-β-D-arabinofuranosyl)-6-N-benzoyladenine

A dry 5 L flask was flushed with dry $N_2$ and charged with THF (1.9 L freshly distilled from lithium aluminum hydride) and $N^6$-benzoyladenine (111.0 g, 0.46 mol). To the well stirred suspension was added NaH (14.0 g, 0.49 mol) as an 80% mineral oil dispersion in one portion. The resulting suspension was heated at reflux under a blanket of $N_2$ for 3 h. A solution of 1-bromo-2-deoxy-3,5-di-O-benzoyl-2-fluoro-α-D-arabinofuranose (186.94 g, 0.44 mol) in THF (350 mL) was added over 2 min from a dropping funnel which was rinsed with THF (150 mL). After refuluxing 3 h the mixture was cooled to 20°C and Celite (20 g) added. The mixture was filtered and the filter washed with EtOAc (4 x 50 mL). The combined filtrate was concentrated to near dryness and redissolved in EtOAc (3.8 L). The organic phase was washed with $H_2O$ (2 x 1 L) then brine (1 x 1 L), dried ($MgSO_4$), filtered, and concentrated. Drying in vacuo to constant weight gave 272.3 g of product 9-(2-deoxy-3,5-di-O-benzoyl-2-fluoro-β-D-arabinofuranosyl)-6-N-benzoyladenine as a foam. The β/α ratio was estimated by NMR to be 15/1. The material was used without further purification: $^1$H NMR (DMSO-$d_6$, major component) δ 4.6-4.9 (m, 3H, C4′, C5′), 5.85 (dq, 1H, C2′), 6.02 (dq, 1H, C3′), 6.80 (dd, 1H, Cl′), 7.3- 8.2 (m, 15H, aromatics), 8.53 (s, 1H, C2), 8.75 (d, 1H, C8).

Procedure 10:

9-(2-Deoxy-2-fluoro-β-D-arabinofuranosyl)adenine.

A flask containing 9-(2-deoxy-3,5-di-O-benzoyl-β-D-arabinofuranosyl)-6-N-benzoyladenine (272.30 g, .44 mol) was charged with MeOH (2 L). The resulting oily suspension was stirred under dry $N_2$ and a solution of NaOMe (.47 mol) in MeOH (600 mL), previously prepared, by dissolving 10.8g. of freshly cut sodium in 600 mL MeOH was added in one portion. The resulting solution was heated at reflux for 2.5 h, then cooled to ca 20°C. The pH was adjusted to 6.5 by portionwise addition of Dowex XB-200 ion-exchange resin (231.26 g). The resin was removed by filtration and washed with MeOH (4 x 100 mL). The filtrate was concentrated to an oil and the product stirred vigorously for 30 min with $H_2O$ (500 mL) and $CH_2Cl_2$ (750 mL). A solid formed and was collected by filtration, washed with $CH_2Cl_2$ (5 x 50 mL) and cold EtOH (4 x 50 mL), and dried to give 68.66 g (57.7% over the two steps) of 9-(2-deoxy-2-fluoro-β-D-arabinofuranosyl)adenine: mp 225-227°C (reported mp 232-234°C, Wright, et al., loc. cit.): $^1$H NMR (DMSO-$d_6$) δ 3.7 (m, 2H, C5′), 3.9 (m, 1H, C4′), 2.45 (dq, 1H, C3′), 5.1 (s, 1H, OH), 5.2 (dt, 1H, C2′), 6.0 (d, 1H, OH), 6.2 (dd, 1H, C1′), 7.38 (s, 2H, $NH_2$), 8.15 (s, 1H, C2), 8.25 (d, 1H, C8).

Procedure 11:

9-(5-O-tert-Butyldimethylsilyl-2-deoxy-2-fluoro-β-D-arabinofuranosyl)adenine.

A mixture of 9-(2-deoxy-2-fluoro-β-D-arabinofuranosyl)adenine (62.0 g, 0.23 mol) and 41.76 g (0.61 mol) of imidazole in dry DMF (310 mL) was stirred and tert-butyldimethylsilyl chloride (40.8 g, 0.27 mol) added. After 50 min, HPLC analysis of the mixture indicated the reaction was incomplete. Additional tert-butyldimethylsilyl chloride (4.83 g, 0.03 mol) was added. After 5 min MeOH (40 mL) was added dropwise, and the resulting mixture poured into EtOAc (1.1 L) and $H_2O$ (1.4 L). The layers were separated and the aqueous layer was extracted with EtOAc (2 x 450 mL). The combined organic extracts were washed with $H_2O$ (3 x 600 mL), brine (1 x 600 mL), then dried ($Na_2SO_4$), and concentrated to a thick precipitate. The solid was slurried with EtOAc (70 mL) and hexane (700 mL) for 25 min and collected. The solid was washed with hexane (2 x 200 mL) and dried in vacuo at 50°C to afford 63.94 g (78.5%) of 9-(5-0-tert-butyldimethylsilyl-2-deoxy-2-fluoro--β-D-arabinofuranosyl)adenine: mp 184-185°C, $^1$H NMR (DMSO-$d_6$) δ 0.08 (2s, 6H, $Si(Me)_2$), 0.90 (s, 9H, $SiC(Me)_3$), 3.8-3.9 (m, 3H, C4′, C5′), 4.42 (dq, 1H, C3′), 5.25 (dt, 1H, C2′), 6.41 (dd, 1H, Cl′), 8.15 (m, 2H, C2, C8).

Procedure 12:

9-(5-O-tert-Butyldiphenylsilyl-2-deoxy-2-fluoro-β-D-arabinofuranosyl)adenine.

A suspension of 9-(2-deoxy-2-fluoro-β-D-arabinofuranosyl)adenine (2.7 g, .01 mol), imidazole (1.58 g, .023 mol) and DMF (16 mL) was stirred and tert-butyldiphenylsilyl chloride (2.76 g, .02 mol) was added. There was a mild exotherm and a complete solution resulted. HPLC analysis indicated that 10% of the starting material remained. Additional tert-butyldiphenylsilyl chloride (.423 g, .0015 mol) was added and after 10 min HPLC analysis indicated only 1% of the starting material remained. MeOH (3.5 mL) was added and the reaction poured into $H_2O$ (200 mL) and extracted with EtOAc (3 x 20 mL). The combined extracts were washed with $H_2O$ (3 x 20 mL) and brine (20 mL), dried ($Na_2SO_4$),and concentrated to a solid. The solid was slurried in a mixture of $Et_2O$ (35 mL) and hexane (50 mL), then filtered and dried, giving 4.8 g (94%): $^1H$ HMR (DMSO-$d_6$) δ 1.0 (s, 9H, SiC(Me)$_3$), 3.9-4.1 (m, 3H, C4′, C5′), 4.6 (dq, 1H, C3′), 5.3 (dt. 1H, C2′), 6.1 (d, 1H, OH), 6.5 (dd, 1H, C1′), 7.31-7.57 (m, 8H, ArH), 7.6-7.7 (m, 4H, ArH), 8.04 (d, 1H, C8), 8.35 (s, 1H, C2).

Procedure 13:

9-(5-O-tert-Butyldiphenylsilyl2-deoxy-2-fluoro-3-O-methylthiothionocarbonyl-β-D-arabinofuranosyl)adenine.

A mixture of 9-(2-deoxy-2-fluoro-5-O-tert-butyldiphenylsilyl-β-D-arabinofuranosyl)adenine (4.29 mg, 8.45 mmol), DMSO (30 mL) and $CS_2$ (1.0 mL, 17.0 mmol) was stirred at 15°C and 5 M NaOH (1.9 mL, 9.5 mmol) was added dropwise. After 30 min MeI (0.92 mL, 9.32 mmol) was added dropwise and a complete solution obtained. HPLC analysis of the reaction indicated that there was no starting material remaining and the reaction was poured into $H_2O$ (200 mL). The solid which formed was filtered and washed with hexane (50 mL) yielding 5.3 g, and used in the next step without further purification. A 0.56 g portion of the material was chromatographed on silica gel (70-200 mesh) eluting with EtOAc, providing 0.38 g of pure xanthate ester. $^1H$ NMR ($CDCl_3$) δ 1.05 (s, 9H, SiC(Me)$_3$),2.6 (s, 3H, SMe), 3.95-4.1 (m, 2H, C5′), 4.25-4.35 (m, 1H, C4′), 5.25 (dd, 1H, C2′), 6.27-6.45 (m, 4H, C1′,C3′ $NH_2$), 7.28-7.5 (m, 6H, ArH), 7.6-7.76 (m, 4H, ArH), 8.04, (d, 1H, C8), 8.35 (s, 1H, C2).

Procedure 14:

9-(5-O-tert-Butyldimethylsilyl-2-deoxy-2-fluoro-3-O-methylthiothiono carbonyl-β-D-arabinofuranosyl)adenine.

A mixture of 9-(5-O--tert-butyldimethylsilyl-2-deoxy-2-fluoro-β-D-arabinofuranosyl)adenine (13.3 g, 34.7 mmol), DMSO (59 mL) and $CS_2$ (4.2 mL, 0.07 mol) was stirred at 15°C and 5M NaOH( 7.72 ml, 38.6 mmol) added dropwise. The reaction mixture was stirred for 30 min and MeI (2.39mL, 38.5 mmol) added dropwise. After addition, HPLC analysis indicated the reaction mixture contained about 3% of the starting material. Additional MeI (0.4 mL, 6.43 mmoles) was added and an HPLC analysis indicated about 2% starting material still remained. Additional MeI (0.2 mL, 3.22 mmol) was added. The mixture was poured into $H_2O$ (600 mL), and after 20 min the yellow solid was filtered, washed with hexane (100 mL), and dried. The weight of product was 18.0 g and it was used without further purification. $^1H$ NMR ($CDCl_3$) δ 0.05 (s, 3H, SiMe), 0.07 (s, 3H, SiMe), 0.85, (s,9H, SiC(Me)$_3$), 2.54 (s, 3H, SMe), 3.85-4.1 (m, 2H, C5′), 4.2-4.3 (m, 1H, C4′), 5.2 (dd, 1H, C2′), 6.03 (s, 2H, $NH_2$), 6.2 (dd, 1H, C3′), 6.47 (dd, 1H, C1′) 8.07 (d, 1H, C8), 8.3 (s, 1H, C2).

Procedure 15:

9-(5-O-tert-Butyldimethylsilyl-2,3-dideoxy-2-fluoro-β-D-arabinofuranosyl)adenine from the corresponding crude xanthate ester.

A suspension of the crude xanthate ester prepared as in Procedure 14 (16.95 g, 35.8 mmol) and toluene (366 mL) was stirred and $N_2$ bubbled into the mixture for 30 min to achieve deoxygenation. Then 2,2′-azobisisobutyronitrile (500 mg, 3.3 mmol) and tri-n-butyltin hydride (14.6 mL, 54.3 mmol) were added successively. The reaction mixture was heated at 80°C for 30 min, then cooled to 0-5°C for 30 min and the

product which crystallized was filtered yielding 22.33 g (92%). $^1H$ NMR (DMSO-$d_6$,)-δ0.08 (2S,6H, SI$(Me)_2$) 0.90 (S,9H, SiC$(Me)_3$), 2.2-2.6, (m,2H, C3'), 3.75 (m,2H,CT), 4.2 (m,1H, C4'), 5.45 (dq, 1H, $J_{H,F}$=56.2 Mz, C2'), 6.35 (dd, 1H, C1'), 7.36 (S, 2H, $NH_2$), 8.18 (S, 2H, C2, C8).

Procedure 16:

9-(5-O-tert-Butyldiphenylsilyl-2,3-dideoxy-2-fluoro-β-D-arabinofuranosyl)adenine from the corresponding crude xanthate ester.

A slurry of crude xanthate (4.68 g, 7.83 mmol) in toluene (80 mL) was purged with $N_2$ for 30 min to achieve deoxygenation. Then 2,2'-azobisisobutyronitrile (175 mg, 1.17 mmol) and tri-n-butyltin hydride (3.2 mL, 11.9 mmol) were added. The mixture was heated at 80°C for 20 min then cooled to 0-5°C for 30 min and the solid product was collected by filtration. The filtrate was concentrated to 15 mL and diluted with 15 mL of hexane to give a second crop. The combined solids were washed with 50% toluene in hexane (50 mL) and hexane (150 mL), and then dried in vacuo giving 2.78 g (72.7%). $^1H$ NMR ($CDCl_3$) δ 1.1 (s, 9H, SiC$(Me)_3$), 2.4-2.7 (m, 2H, C3'), 3.85 (d, 2H, C5'), 4.25 (dq, 1H, C2'), 4.35 (m, 1H, C4'), 6.0 (s, 2H, $NH_2$), 6.3 (dd, 1H, C1'), 7.3-7.5 (m, 6H, ArH), 7.6-7.8 (m, 2H, ArH), 8.05 (d, 1H, C2), 8.35 (s, 1H, C8).

Procedure 17:

9-(2,3-Dideoxy-2-fluoro-β-D-arabinofuranosyl)adenine. FddaraA

A solution of 9-(5-O-tert-butyldimethylsilyl-2,3-dideoxy-2-fluoro-β-D-arabinofuranosyl)adenine (2.5 g, 6.8 mmol) and THF (25 mL), was stirred and 1M tetra-butylammonium fluoride ( 7.48 mL, 7.48 mmol) in THF was added dropwise. After 20 min the solvent was evaporated and the residue diluted with cold EtOH (10 mL). The resulting solution was stirred and crystals formed, which were filtered and dried giving 1.7 g (98%): $^1H$ NMR (DMSO-$d_6$) δ 2.2-2.6 (m, 2H, C3'), 3.8 (m, 2H, C5'), 4.2 (m, 1H, C4'), 5.1 (t, 1H, OH), 5.4 (m, 1H, $J_{H,F}$=58 Hz, C2'), 6.3 (dd, 1H, C1'), 7.35 (s, 2H, $NH_2$), 8.16 (s,1H, C2), 8.25 (d 1H, C8).

Procedure 18:

9-(2,3-Dideoxy-2-fluoro-β-D-arabinofuranosyl)hypoxanthine from 9-(2,3-dideoxy-2-fluoro-β-D-arabinofuranosyl)adenine FddaraA.

A slurry of 9-(2,3-dideoxy-2-fluoro-β-D-arabinofuranosyl)adenine, (6.2 g, 24.5 mmol), $H_2O$ (300 mL) and adenosine deaminase (110 mg, Sigma type II crude powder from calf intestinal mucosa) was stirred at 36°C for 16 h. Conversion was judged complete by HPLC analysis and the solution was evaporated under reduced pressure to give 6.35 g of white crystals. The pure material was obtained by flash chromatography on silica gel (eluting with 5-10% MeOH-$CH_2Cl_2$)yielding 5.3 g (85%) of product. NMR data is the same as previously reported.

Procedure 19:

9-(5-O-Benzoyl-2-deoxy-2-fluoro-β-D-arabinofuranosyl)-6-chloropurine:

A mixture of 9-(3,5-di-O-benzoyl-2-deoxy-2-fluoro-β-D-arabinofuranosyl)-6-chloropurine (1.0 g, 2.01 mmol) in anhyd. THF (12 mL) and MeOH (7.5 mL) was stirred at room temperature for 23 h. The mixture was filtered and the filtrate was evaporated. The resulting product was redissolved in EtOAc (20 mL) and washed with water and brine. It was dried ($Na_2SO_4$) and evaporated to give the crude product (1.03 g). Purification over silica gel (70-230 mesh) using hexane-EtOAc (2:1 and 1:1) afforded the title nucleoside

(0.609 g, 80.3%) as a white solid. $^1H$ NMR ($CDCl_3$): δ 3.97 (d, 1H, OH), 4.39-4.51 (m, 1H, C4′), 4.57-4.85 (m 3H, C3′, C5′), 5.17 (md, 1H, C2′), 6.63 (dd, 1H, C1′), 7.39 (t, 2H, Ar-H), 7.52 (t, 1H, Ar-H), 7.96 (d, 2H, Ar-H), 8.36 (d, 1H, C8), 8.68 (s, 1H, C2).

Repeating the above experiment at 60°C for 1 3/4 h furnished three nucleosides, 9-(5-O-benzoyl-2-deoxy-2-fluoro-β-D-arabinofuranosyl)-6-chloropurine (71.4%), 9-(5-O-benzoyl-2-deoxy-2-fluoro-β-D-arabinofuranosyl)-6-methoxypurine (2.8 %) and 9-(2-deoxy-2-fluoro-β-D-arabinofuranosyl)-6-chloropurine (15.4%).

Procedure 20:

9-(5-O-Benzoyl-2-deoxy-2-fluoro-3-O-methylthiothionocarbonyl-β-D-arabinofuranosyl)-6-chloropurine:

A solution of 9-(5-O-benzoyl-2-deoxy-2-fluoro-β-D-arabinofuranosyl)-6-chloropurine (0.241 g, 0.64 mmol) and $CS_2$ (0.078 mL, 1.3 mmol) in DMSO (1.5 mL) was cooled to 15°C and treated with 5 M NaOH (0.14 mL, 0.72 mmol). The resulting yellow solution was stirred for 20 min. MeI (0.044 mL, 0.71 mmol) was added and stirred for 10 min. The solution was poured into water (20 mL) and extracted with EtOAc (2 x 20 mL). The organic phase was washed with water (5 x 10 mL) and brine (10 mL), dried ($Na_2SO_4$) and evaporated to furnish yellowish gummy material (0.320 g), which contained 8% of 9-(3-O-benzoyl-2-deoxy-2-fluoro-5-methylthiothionocarbonyl-β-arabinofuranosyl)-6-chloropurine and 92% of the required nucleoside based on its NMR analysis.

Chromatographic purification of the above product over a silica gel (70-230 mesh) column using hexane-EtOAc (2:1) gave the pure xanthate ester (0.257 g, 86%) as a colorless solid. $^1H$ NMR ($CDCl_3$): δ 2.63 (s, 3H, $SCH_3$), 4.59-4.67 (m, 1H, C4′), 4.70-4.87 (m, 2H, C5′), 5.34 (dd, 1H, C2′) 6.26 (dd, 1H, C3′), 6.64 (dd, 1H, C1′), 7.41 (t, 2H, Ar-H), 7.53 (t, 1H, Ar-H), 8.05 (dd, 2H, Ar-H), 8.36 (d, 1H, C8), 8.73 (s, 1H, C2).

Procedure 21:

9-(5-O-Benzoyl-2,3-dideoxy-2-fluoro-β-D-arabinofuranosyl)-6-chloropurine:

Nitrogen gas was bubbled into a solution of 9-(5-O-benzoyl-2-deoxy-2-fluoro-3-methylthiothionocarbonyl-β-D-arabinofuranosyl)-6-chloropurine * (0.775 g, 1.66 mmol) in anhyd. toluene (18 mL) for 30 min. Then 2,2′-azobisisobutyronitrile (0.038 g, 0.23 mmol) and tributyltin hydride (0.679 mL, 2.52 mmol) were added successively and the reaction was heated at 80°C under $N_2$. The reaction was cooled and the toluene was evaporated to ca. 2 mL. This was swirled with hexane (3 mL) and the hexane was decanted to remove tin compounds. The rest of the product was applied to a silica gel (70-230 mesh) column, which was eluted with hexane-EtOAc (3:1). This afforded 9-(3-O-benzoyl-2-deoxy-2-fluoro-5-O-methylthiothionocarbonyl-β-D-arabino- furanosyl)-6-chloropurine (29 mg, 3.7%) and the title nucleoside (0.5 g, 80%): $^1$ H NMR ($CDCl_3$: δ 2.45-2.88 (m, 2H, C3′), 4.49-4.9 (m, 3H, C4′ and C5′), 5.33 (dd, 1H, C2′), 6.42 (dd, 1H, C1′), 7.34-7.73 (m, 3H, Ar-H), 8.05 (d, 2H, Ar-H), 8.43 (d, 1H, C8), 8.72 (s, 1H, C2).

Procedure 22.

9-(2,3-Dideoxy-2-fluoro-β-D-arabinofuranosyl)adenine (FddaraA)

Saturated methanolic ammonia (20 mL) was added to 9-(5-O-benzoyl-2,3-dideoxy-2-fluoro-β-D-arabinofuranosyl)-6-chloropurine (200 mg, 0.53 mmol) at 0° C in a steel bomb, followed by heating at 83° C for 19 h. After cooling to room temperature, the resulting solution was evaporated to give an off-white solid (195 mg). This was triturated with diethyl ether (5 mL) and the insoluble material was collected on a filter

*This contains 3.5% of its regio isomer, 9-(3-O-benzoyl-2-deoxy-2-fluoro-5-O-methylthiothionocarbonyl-β-D-arabinofuranosyl)-6-chloropurine.

and then heated at 85° C with absolute ethanol. The resulting crystalline slurry was stored at 0-5° C overnight. The solids were collected by suction filtration, washed with absolute ethanol (0.5 mL) and diethyl ether (1 mL), and dried in vacuo to constant weight to give 93 mg (69.4%) of FddaraA.

The filtrate was concentrated to dryness and was chromatographed over a column of silica gel (70-230 mesh), eluting with 10% methanol in dichloromethane. Concentration of the appropriate fractions gave benzamide (26.5 mg), 9-(2,3-dideoxy-2-fluoro-β-D-arabinofuranosyl)-6-methoxypurine (16 mg, 7.8% and FddaraA (10.5 mg, 7.8%). The combined total yield of FddaraA was 103.5 mg (77.2%). The NMR spectrum of this material was identical to material previously obtained.

Procedure 23.

9-(2,3-Dideoxy-2-fluoro-β-D-arabinofuranosyl)hyopoxanthine (Fddaral)

2 M sodium hydroxide (0.41 mL, 0.82 mmol) was added to 9-(5-O-benzoyl-2,3-dideoxy-2-fluoro-β-D-arabinofuranosyl)-6-methoxypurine (59 mg, 0.16 mmol) and the resulting solution was heated at 80° C for 2.5 h. After this time, HPLC analysis indicated the reaction was complete. The solution was cooled, neutralized with Dowex x8-200 ion-exchange resin (H+ form) and filtered through a pad of Celite. The pad was washed with water (5 mL) and methanol (20 mL). The combined filtrates were concentrated to a yellow solid which was purified by chromatography over silica gel (70-230 mesh) using 5-10% methanol in dichloromethane as eluant and gave a total of 31 mg (77.9%) of Fddaral. The NMR spectrum of this material was identical to material previously obtained.

## Claims

1. The process for preparing an intermediate useful for preparing 9-(2,3-dideoxy-2-fluoro-β-D-arabinofuranosyl)hypoxanthineor 9-(2,3-dideoxy-2-fluoro-β-D-arabinofuranosyl)adenine said intermediate having Formula III

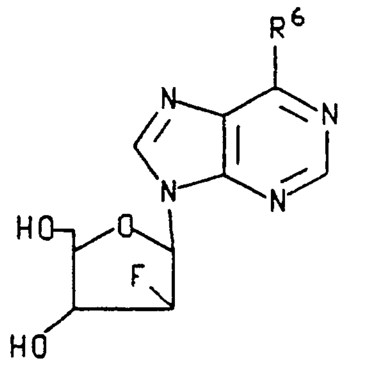

(III)

wherein $R_6$ is methoxy or amino which comprises

(a) forming the sodium salt of a purine reactant selected from 6-chloropurine and 6-benzoylaminopurine and contacting therewith in a solvent and under anhydrous conditions an equimolar amount of 1-bromo-2-deoxy-3,5-di-O-benzoyl-2-fluoro-α-D-arabinofuranose at a temperature in the range of 20-90° C until the said arabinofuranose reactant is consumed;

(b) heating the product of step (a) at the reflux temperature in methanol solution in a dry atmosphere with about 1.1 molecular proportions of sodium methoxide until conversion thereof to the intermediate of Formula III is complete.

2. The process of Claim 1 wherein 6-chloropurine is the reactant in step (a) and the solvent is a mixture of approximately equal volumes of acetonitrile and methylene chloride.

3. The process of Claim 1 wherein $N^6$-benzoyladenine is the reactant in step (a) and the solvent is tetrahydrofuran.

4. The process for preparing 9-(2,3-dideoxy-2-fluoro-β-D-arabinofuranosyl)adenine which comprises

(c) converting the 5′-hydroxyl group of the intermediate of Formula III of Claim 1 wherein $R^6$ is amino to

the tert-butyldi-$R^2$-silyl protecting group wherein $R^2$ is methyl or phenyl by contacting said intermediate of Formula III with a substantially equimolar amount of tert-butyldimethylsilyl chloride or tert-butyldiphenylsilyl chloride under reaction conditions and isolating the resulting protecting group containing product in purified form;

(d) forming the 3′-methylthiothionocarbonyl ester of the product of step (c) and reducing said ester by contacting with azobisisobutyronitrile and tri-n-butyltin hydride under reaction conditions thereby producing the intermediate of Formula V wherein $R^6$ is $NH_2$ and $R^2$ is as defined above;

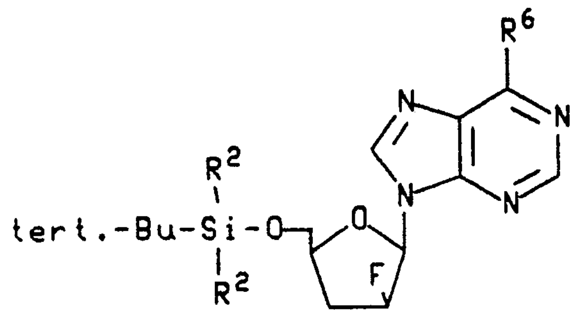

V

(e) replacing the tert-butyldi-$R^2$-silyl protecting group of the intermediate of Formula V with hydrogen thereby providing the desired adenine product.

5. The process for preparing 9-(2,3-dideoxy-2-fluoro-β-D-arabinofuranosyl)hypoxanthine which comprises

(c) converting the 5′-hydroxyl group of the intermediate of Formula III of Claim 1 wherein $R^6$ is methoxy to the tert-butyldi-$R^2$-silyl protecting group wherein $R^2$ is methyl or phenyl by contacting said intermediate with a substantially equimolar amount of tert-butyldimethylsilyl chloride or tert-butyldiphenylsilyl chloride under reaction conditions and isolating the resulting protecting group containing product in purified form;

(d) forming the 3′-methylthiothionocarbonyl ester of the product of step (c) and reducing said ester by contacting with azobisisobutyronitrile and tri-n-butyltin hydride under reaction conditions, thereby producing the intermediate of Formula V wherein $R^6$ is methoxy and $R^2$ is as defined above,

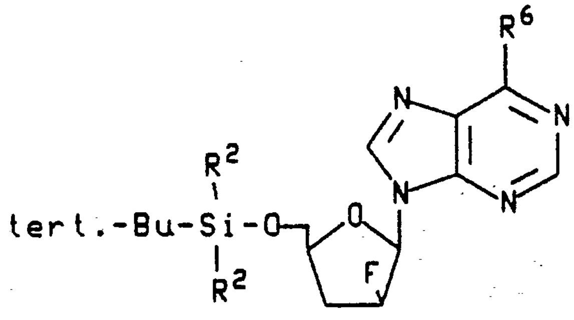

V

(e) replacing the tert-butyldi-$R^2$-silyl protecting group and the 6-O-methyl group of the intermediate of Formula V with hydrogen thereby providing the desired hypoxanthine product.

6. The process of Claim 4 or Claim 5 wherein in step (c) the intermediate of Formula III is dissolved in dry dimethylformamide and the solution is treated with at least 2 molecular proportions of imidazole and then with sufficient tert-butyldimethylsilyl chloride or tert-butyldiphenylsilyl chloride during a period of time sufficient to complete the formation of the 5′-tert-butyldimethylsilyl or 5′-tert-butyldiphenylsilyl ether.

7. The process of anyone of Claims 4, 5 or 6 wherein in step (d) the product of step (c) is dissolved in dimethylsulfoxide and treated with more than one molecular proportion of carbon disulfide at 15° C, and thereafter the mixture is treated in sequence with an equimolar amount of aqueous sodium hydroxide and methyl iodide thereby forming said methylthiothionocarbonyl ester.

8. The process of anyone of Claims 4 to 7 wherein in step (d) reducing said ester is carried out in toluene solution at 80° C.

9. The process of anyone of Claims 5 to 8 wherein in step (e) heating with aqueous 2N NaOH at 75° C is employed.

10. The process of anyone of Claims 5 to 8 wherein step e) is carried out in two steps comprising (f) contacting the product of step (d) first with trimethylsilyl bromide or trimethylsilyl iodide thereby removing the 6-O-methyl group, and thereafter contacting with tetra-n-butylammonium fluoride thereby removing the 5′-O-tert-butyldi-$R^2$-silyl group.

11. The process of Claim 10 wherein in step (f) dry acetonitrile is employed as solvent and an approximately equimolar amount of trimethylsilyl iodide is employed at a temperature in the range of 20-50 °C for sufficient time to complete the reaction.

12. The process of Claim 10 wherein in step (e) tetrahydrofuran is employed as solvent during contacting with tetra-n-butylammonium fluoride for a time period sufficient for completion of the reaction.

13. The process of anyone of Claims 4 or 6 to 8 wherein in step (e)the intermediate of Formula V is contacted with approximately an equimolar amount of tetra-n-butylammonium fluoride in tetrahydrofuran solution for a sufficient time to replace the 5′-O-tert-butyldi-$R^2$-silyl group with hydrogen.

14. The process for preparing 9-(2,3-dideoxy-2-fluoro-β-D-arabinofuranosyl)hypoxanthine employing the intermediate of Formula III

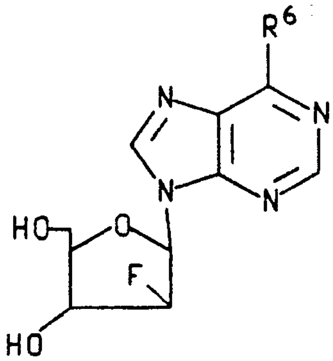

(III)

wherein $R^6$ is methoxy which process comprises

(a) forming the sodium salt of 6-chloropurine as purine reactant and contacting therewith in a solvent and under anhydrous conditions an equimolar amount of 1-bromo-2-deoxy-3,5-di-O-benzoyl-2-fluoro-α-D-arabino furanose at a temperature in the range of 20-90 °C until the said arabinofuranose is consumed;

(b) heating the product of step (a) at the reflux temperature in methanol solution in a dry atmosphere with about 1.1 molecular proportions of sodium methoxide until conversion thereof to the said intermediate of Formula III is complete;

(c) converting the 5′-hydroxyl group of the said intermediate of Formula III to the tert-butyldi-$R^2$-silyl protecting group wherein $R^2$ is methyl or phenyl by contacting said intermediate with a substantially equimolar amount of tert-butyldimethylsilyl chloride or tert-butyldiphenylsilyl chloride under reaction conditions and isolating the resulting protecting group containing product in purified form;

(d) forming the 3′-methylthiothionocarbonyl ester of the product of step (c) and reducing said ester by contacting with azobisisobutyronitrile and tri-n-butyltin hydride under reaction conditions thereby producing the intermediate of Formula V wherein $R^6$ is methoxy and $R^2$ is as defined above.

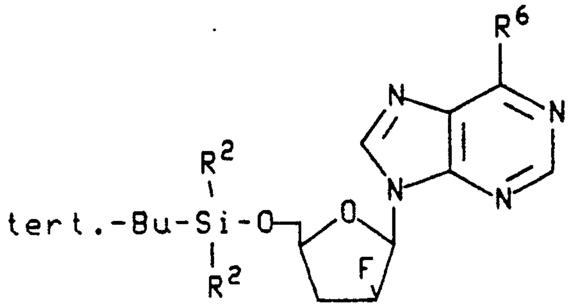

V

(e) replacing the tert-butyldi-$R^2$-silyl protecting group and the 6-O-methyl group of the intermediate of Formula V with hydrogen thereby providing the desired hypoxanthine product.

15. The process for preparing 9-(2,3-dideoxy-2-fluoro-β-D-arabinofuranosyl)adenine employing the intermediate of Formula III

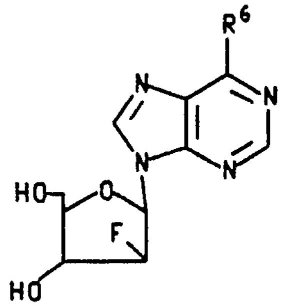

(III)

wherein $R^6$ is amino which comprises

(a) forming the sodium salt of 6-benzoylaminopurine as purine reactant and contacting therewith in a solvent and under anhydrous conditions an equimolar amount of 1-bromo-2-deoxy-3,5-di-O-benzoyl-2-fluoro-α-D-arabinofuranose at a temperature in the range of 20-90°C until the said arabinofuranose reactant is consumed;

b) heating the product of step (a) at the reflux temperature in methanol solution in a dry atmosphere with about 1.1 molecular proportions of sodium methoxide until conversion thereof to the said intermediate of Formula III is complete;

(c) converting the 5′-hydroxyl group of the said intermediate of Formula III to the tert-butyldi-$R^2$-silyl protecting group wherein $R^2$ is methyl or phenyl by contacting said intermediate with a substantially equimolar amount of tert-butyldimethylsilyl chloride or tert-butyldiphenylsilyl chloride under reaction conditions and isolating the resulting protecting group containing product in purified form;

(d) forming the 3′-methylthiothionocarbonyl ester of the product of step (c) and reducing said ester by contacting with azobisisobutyronitrile and tri-n-butyltin hydride under reaction conditions thereby producing the intermediate of Formula V wherein $R^6$ is amino, and $R^2$ as defined above,

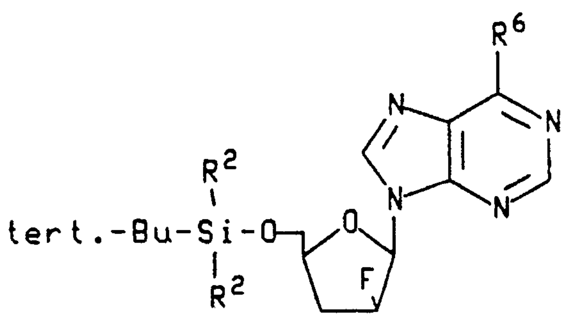

V

(e) replacing the tert-butyldi-$R^2$-silyl protecting group of the intermediate of Formula V with hydrogen thereby providing the desired adenine product.

16. 9-(2-Deoxy-3,5-di-O-benzoyl-2-fluoro-β-D-arabinofuranosyl)-6-chloropurine;

9-(5-O-tert-Butyldi-$R^2$-silyl-2-deoxy-2-fluoro-β-D-arabinofuranosyl)-6-methoxypurine wherein $R^2$ is methyl or phenyl;

9-(2-Deoxy-2-fluoro-3-O-methylthiothionocarbonyl-5-O-tert-butyldi-$R^2$-silyl-β-D-arabinofuranosyl)-6-methoxypurine wherein $R^2$ is methyl or phenyl;

9-(2-Deoxy-2-fluoro-3-O-methylthiothionocarbonyl-5-O-tert.-butyldi-$R^2$-silyl-β-D-arabinofuranosyl)adenine wherein $R^2$ is methyl or phenyl;

9-(5-O-tert-butyldi-$R^2$-silyl, 2,3-dideoxy-2-fluoro-β-D-arabinofuranosyl)-6-methoxypurine wherein $R^2$ is methyl or phenyl ;

9-(5-O-tert-Butyldi-$R^2$-silyl-2,3-dideoxy-2-fluoro-β-D-arabinofuranosyl)hypoxanthine, wherein $R^2$ is methyl or phenyl;

9-(5-O-Benzoyl-2-deoxy-2-fluoro-β-D-arabinofuranosyl)-6-chloropurine;

9-(5-O-Benzoyl-2,3-dideoxy-2-fluoro-β-D-arabinofuranosyl)-6-chloropurine;

9-(5-O-Benzoyl-2-deoxy-2-fluoro-3-O-methylthiothiocarbonyl-β-D-arabinofuranosyl)-6-chloropurine; and 9-(2,3-dideoxy-2-fluoro-β-D-arabinofuranosyl)-6-methoxypurine.

17. The process for preparing an intermediate useful for preparing 9-(2,3-dideoxy-2-fluoro-β-D-arabinofuranosyl)hypoxanthineor 9-(2,3-dideoxy-2-fluoro-β-D-arabinofuranosyl)adenine said intermediate having Formula VII

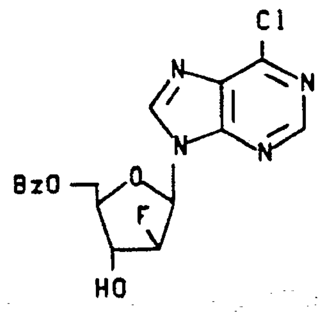

VII

wherein Bz is the benzoyl group which comprises

(a) forming the sodium salt of 6-chloropurine as purine reactant and contacting therewith in a solvent and under anhydrous conditions an equimolar amount of 1-bromo-2-deoxy-3,5-di-O-benzoyl-2-fluoro-α-arabinofuranose at a temperature in the range of 20-90°C until the said arabinofuranose is consumed, recovering the 9-(2-deoxy-3,5-di-O-benzoyl-2-fluoro-β-D-arabinofuranosyl)-6-chloropurine thereby produced, and (g) contacting the product of Step (a) with sodium bicarbonate in a tetrahydrofuran/methanol anhydrous solvent mixture at room temperature during a time period sufficient to selectively replace said 3-O-benzoyl group with hydrogen.

18. The process for preparing 9-(2,3-dideoxy-2-fluoro-β-D-arabinofuranosyl)hypoxanthine which comprises

(a) forming the sodium salt of 6-chloropurine as purine reactant and contacting therewith in a solvent and under anhydrous conditions an equimolar amount of 1-bromo-2-deoxy-3,5-di-O-benzoyl-2-fluoro-α-D-arabinofuranose at a temperature in the range of 20-90°C until the said arabinofuranose is consumed, recovering the 9-(3,5-di-O-benzoyl-2-deoxy-2-fluoro-β-D-arabinofuranosyl)-6-chloropurine thereby produced, and (g) contacting the product of Step (a) with sodium bicarbonate in a tetrahydrofuran/methanol anhydrous solvent mixture at room temperature during a time period sufficient to selectively replace said 3-O-benzoyl group with hydrogen, thereby forming the intermediate of Formula VII.

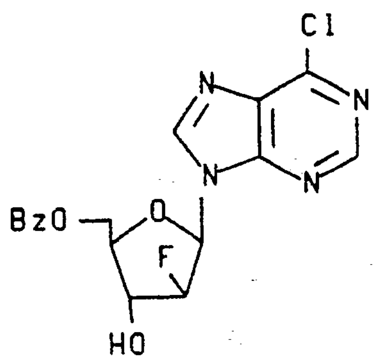

VII

(d) forming the 3′-methylthiothionocarbonyl ester of the product of step (g) and reducing said ester by contacting with azobisisobutyronitrile and tri-n-butyltin hydride under reaction conditions thereby producing the intermediate of Formula VIII wherein Bz is as defined above;

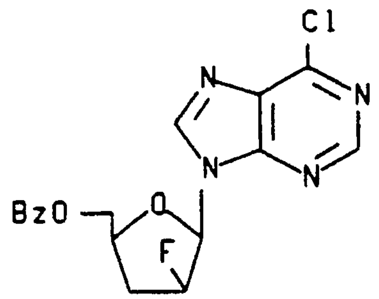

VIII

(b) treating the intermediate of Formula VIII at a temperature in the range of 20-100° C with at least 2 molecular proportions of aqueous sodium hydroxide during a time period sufficient to replace said 5-O-benzoyl group with hydrogen and said 6-Cl group with -OH and yielding the desired hypoxanthine product.

19. The process for preparing 9-(2,3-dideoxy-2-fluoro-β-D-arabinofuranosyl)adenine which comprises (a) forming the sodium salt of 6-chloropurine as purine reactant and contacting therewith in a solvent and under anhydrous conditions as equimolar amount of 1-bromo-2-deoxy-3,5-di-O-benzoyl-2-fluoro-β-D-arabinofuranose at a temperature in the range of 20-90° C until the said arabinofuranose is consumed, recovering the 9-(2-deoxy-3,5-di-O-benzoyl-2-fluoro-β-D-arabinofuranosyl)-6-chloropurine thereby produced, and (g) contating the product of Step (a) with sodium bicarbonate in a tetrahydrofuran/methanol anhydrous solvent mixture at room temperature during a time period sufficient to selectively replace said 3-O-benzoyl group with hydrogen, thereby forming the intermediate of Formula VII ;

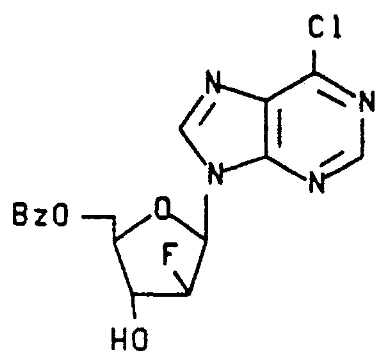

VII

(d) forming the 3′-methylthiothionocarbonyl ester of the product of Step (g) and reducing said ester by contacting with azobisisobutyronitrile and tri-n-butyltin hydride under reaction conditions thereby producing the intermediate of Formula VIII wherein Bz is as defined above:

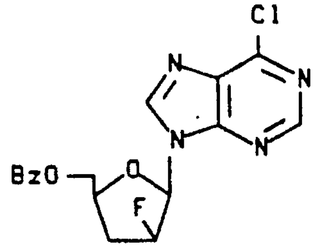

VIII

(i) treating the intermediate of Formula VIII at a temperature in the range of 20-100° C with sufficient saturated methanolic ammonia during a time period sufficient to replace said 5-O-benzoyl group with hydrogen and the said 6-Cl group with $-NH_2$ and yielding the desired adenine product.